(19) Europäisches Patentamt / European Patent Office / Office européen des brevets

(11) **EP 4 347 014 B1**

(12) **EUROPEAN PATENT SPECIFICATION**

(45) Date of publication and mention
of the grant of the patent:
**04.03.2026 Bulletin 2026/10**

(21) Application number: **22731553.8**

(22) Date of filing: **31.05.2022**

(51) International Patent Classification (IPC):
*A61N 5/10* (2006.01)     *A61B 5/00* (2006.01)
*A61B 5/055* (2006.01)

(52) Cooperative Patent Classification (CPC):
**A61N 5/1049; A61B 5/055; A61B 5/4836;
A61B 5/7285; A61B 5/7292; A61N 5/1067;
A61N 5/1068;** A61N 2005/1055

(86) International application number:
**PCT/EP2022/064690**

(87) International publication number:
**WO 2022/253797 (08.12.2022 Gazette 2022/49)**

(54) **DRIVING GATED TREATMENT DELIVERIES**

ANTRIEBSGESTEUERTE BEHANDLUNGSABGABEN

RÉALISATION D'ADMINISTRATIONS DE TRAITEMENTS SYNCHRONISÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.06.2021 GB 202107793**

(43) Date of publication of application:
**10.04.2024 Bulletin 2024/15**

(73) Proprietor: **Elekta Limited
Crawley, West Sussex RH10 9BL (GB)**

(72) Inventor: **FREEDMAN, Joshua
London Road Crawley Sussex RH10 9RR (GB)**

(74) Representative: **Beal, James Michael et al
Kilburn & Strode LLP
Lacon London
84 Theobalds Road
London WC1X 8NL (GB)**

(56) References cited:
**WO-A1-2020/219814     DE-A1- 102013 206 315**

**Description**

This disclosure relates to controlling radiotherapy devices, and in particular to gating of treatment beams in radiotherapy devices.

Background

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises a gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

In radiotherapy treatment, it is desirable to deliver a prescribed dose of radiation to a target region of a subject and to limit irradiation of other parts of the subject, i.e. to healthy tissue. Motion of the subject can cause a decreased dose to be applied to the target region and/or an increased dose to be applied to the healthy tissue. To address this, known techniques include monitoring a location of the subject and gating the treatment beam such that radiation is applied only when the subject (i.e. the target region within the subject) is in a desired location and not when the subject/target region is in a suboptimal location. This improves clinical outcomes.

There are various physiological motions that can contribute to a total motion of a subject. Gross or large-scale movements of a subject may include shifting position, coughing or sneezing. The subject may also undergo cyclical, physiological movement. For example, the subject may undergo respiratory motion due to their breathing cycle. The subject may also undergo cardiac motion based on beating of their heart. These motions can alter the location of a subject and/or of a tumour in a time-dependent manner relative to the respective location of the subject and/or of the tumour at the start of the radiotherapy treatment.

Locations of subject anatomy during a radiotherapy treatment can be determined from MR (magnetic resonance) images. These may be two-dimensional MR images taken in intersecting planes. This provides time-dependent information on the location of the subject and their internal anatomy. However, the acquisition times required to measure these raw MR data may be around 0.2 s. Furthermore performing image reconstruction to derive these MR images from raw MR data, and performing analysis on these images, is computationally intensive and leads to higher than desired gating system latencies. Gating system latencies may be around 0.5 s, meaning that the treatment beam will not be turned off until around 0.5 s after movement of the subject's internal anatomy to an undesirable location. This is suboptimal for respiratory and cardiac cycles and may miss altogether sudden involuntary movements such as those due to coughs or hiccups. Prediction models, such as linear regression filters, can be exploited to reduce gating system latencies. In such approaches, the prediction model is applied to the pre-recorded gating signal (derived from 2D MR images) at the current time to infer the gating signal at a future time. The inferred signal can then be utilised to drive gated treatment deliveries. However, prediction modelling techniques can result in inference errors when applied to irregular respiratory patterns, leading to inaccurate gating decisions.

A disadvantage of using conventional 2D MR images is that only a limited field of view is acquired of the subject. In addition, it is only possible to apply a gradient non-linearity geometrical distortion correction in the plane of the 2D image, because no information in the through plane is available. This limits the geometrical accuracy of the reconstructed images. Furthermore, the geometrical accuracy of conventional 2D MR images is diminished by magnetic susceptibility artefacts, static field inhomogeneities and chemical shift. These limitations restrict the potential for offline dose reconstruction, which is used to estimate the true, delivered dose applied to the subject by re-calculating the prescribed treatment plan on the reconstructed images acquired during treatment delivery. This delivered dose can be used to optimise the dose prescribed in subsequent treatment deliveries. Generating 3D MR images can increase the accuracy of such techniques. However, performing image acquisition and reconstruction to generate 3D MR images, and performing analysis on these images, is even more computationally intensive and further increases latency.

It would be advantageous to provide improved means for accounting for and reacting to changeable subject locations during a radiotherapy treatment. In addition, it would be advantageous to reduce the latency of gating in radiotherapy treatment. It would also be advantageous to improve the anatomical coverage and geometrical accuracy of images taken during radiotherapy treatment.

WO 2020/219814 A1 describes magnetic resonance signature matching (MRSIGMA) for real-time volumetric motion tracking and adaptive radiotherapy. DE 10 2013 206315 A1 describes a method and computing device for supporting radiation therapy treatment.

The present invention seeks to address these and other disadvantages encountered in the prior art.

**EP 4 347 014 B1**

Summary

An invention is set out in the independent claims.

According to an aspect, there is provided a radiotherapy device comprising: a radiation source configured to generate a radiotherapy beam for irradiating a subject; an MR imaging apparatus configured to apply a 3D MR pulse sequence to obtain k-space data for the subject; and a controller communicatively coupled to the radiation source and the MR imaging apparatus, wherein the controller is configured to: select central k-space data from the k-space data; generate a beam gating control signal based on the central k-space data; and, after generating the beam gating control signal, reconstruct an image of the subject based at least in part on the k-space data. According to a further aspect, there is provided a computer-readable medium comprising computer-executable instructions which, when executed by a processor, cause the processor to: apply a 3D MR pulse sequence using an MR imaging apparatus to obtain k-space data for a subject; select central k-space data from the k-space data; generate a beam gating control signal based on the central k-space data; and, after generating the beam gating control signal, reconstruct an image of the subject based at least in part on the k-space data.

Figures

Specific embodiments are now described, by way of example only, with reference to the drawings, in which:

Figure 1 depicts a radiotherapy device or apparatus according to the present disclosure;

Figure 2 depicts a schematic of an MR imaging apparatus according to the present disclosure;

Figures 3a and 3b depict an example dynamic gating window simulation according to the present disclosure;

Figure 4 depicts an example of iterative image reconstruction according to the present disclosure;

Figure 5 depicts a method for extracting a self-gating signal;

Figure 6 depicts another method for extracting a self-gating signal;

Figure 7 depicts a method for integrated MR imaging and radiotherapy delivery according to the present disclosure;

Figure 8 depicts a block diagram of one implementation of a computing device according to the present disclosure.

Detailed Description

The present disclosure relates to controlling radiotherapy devices. During a radiotherapy treatment, the radiotherapy beam may be gated based on a location of a subject or a part of the subject. For example, the radiotherapy beam may be turned off if it is determined, based on one or more images of the subject, that the subject has moved such that healthy tissue may be irradiated with a higher than acceptable dose. To reduce the latency of this gating, the present disclosure enables gating of the radiotherapy beam based on central k-space data obtained by an MR imaging apparatus rather than relying on reconstruction of images of the subject based on k-space data. K-space data is obtained using a 3D MR pulse sequence, i.e. a pulse sequence which has a 3D k-space trajectory, and central k-space data is selected from this k-space data. This central k-space data is used to generate a beam gating control signal for gating the radiotherapy beam. By driving the gating using this central k-space data, the gating latency is reduced. This is because central k-space data can be more rapidly acquired using an MR imaging apparatus and more rapidly analysed by a gating algorithm compared to 2D image data.

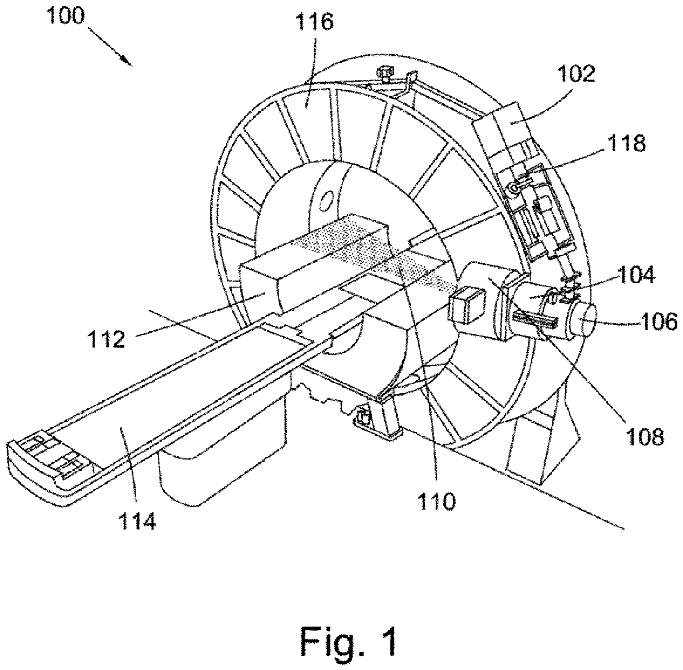

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in Figure 1 is in accordance with the present disclosure and is suitable for use with the disclosed systems and apparatuses. While the device in Figure 1 is an MR-linac, the implementations of the present disclosure may be any radiotherapy device, for example a linac device.

The device 100 depicted in Figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in Figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table.

The radiotherapy apparatus / device depicted in Figure 1 also comprises MR imaging apparatus 112. The MR imaging apparatus 112 is configured to obtain images of a subject positioned, i.e. located, on the patient support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor (also referred to as an MR controller), which controls the MR imaging apparatus 112; an RT apparatus processor, which controls the operation of the RT apparatus; and a patient support surface processor which controls the operation and actuation of the patient support surface 114. The controller is communicatively coupled to a memory, e.g. a computer readable medium. The linac device also comprises several other components and systems as will be understood by the skilled person. For example, in order to ensure the linac does not leak radiation, appropriate shielding is also provided.

The radiotherapy device may be configured to perform any of the non-claimed method steps presently disclosed and may comprise computer executable instructions which, when executed by a processor, cause a processor to perform any of the method steps presently disclosed. Any of the steps that the radiotherapy device is configured to perform may be considered as method steps of the present disclosure and may be embodied in computer executable instructions for execution by a processor.

MR imaging is an imaging technique which involves placing a subject in a strong magnetic field which aligns the magnetic moments of protons in the subject to produce a net magnetization. Irradiating the subject with radiofrequency (RF) pulses of a particular resonant frequency tips the net magnetization of these protons by a flip-angle $\alpha°$ into a plane perpendicular to the strong magnetic field. Immediately after the RF pulse is completed, the tipped net magnetization of these protons realigns with the strong magnetic field. The changing magnetic flux generated during realignment induces a voltage in a coil. This is measured and analysed to provide information on the distribution of different tissues within the subject. While the skilled person will be familiar with MR imaging techniques, the following explanation of the general principles is provided below.

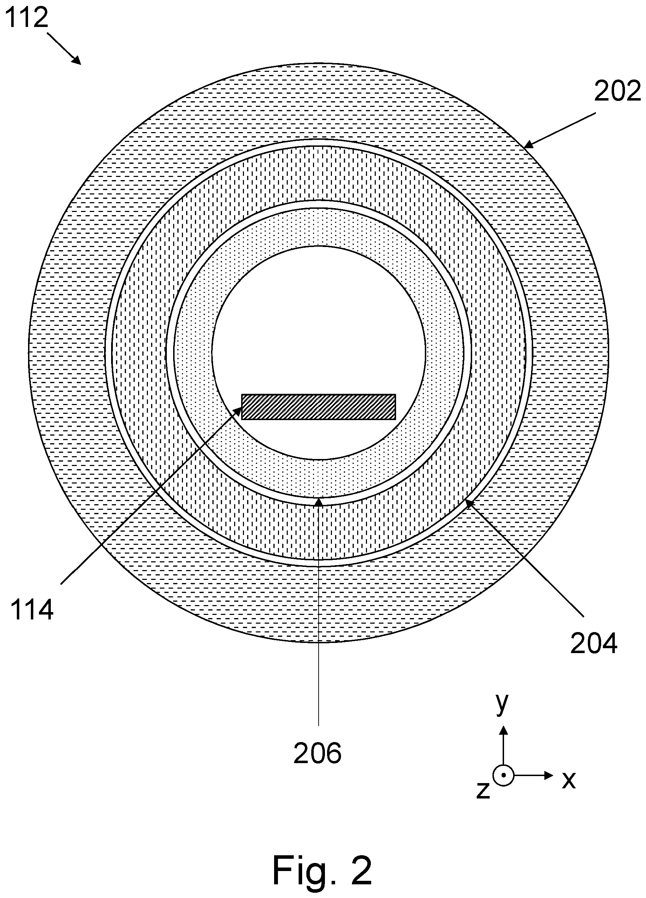

Figure 2 depicts a schematic of an MR imaging apparatus 112. The MR imaging apparatus 112 may be comprised in the device 100. Figure 2 depicts a view of the MR imaging apparatus 112 taken along the bore of the MR imaging device 112 and/or of the gantry 116. This bore is depicted as being oriented along the z-axis. The end of the patient support surface 114 is also depicted in Figure 2. As shown in Figure 2, the MR imaging apparatus 112 comprises a primary magnetic field coil 202, a magnetic gradient coil 204 and a radiofrequency (RF) coil 206. Each of these may have cylindrical symmetry around the bore and each may be positioned concentrically around the bore.

The primary magnetic field coil 202 is configured to produce a constant magnetic field along the bore of the MR imaging apparatus. This constant magnetic field is oriented in the z-direction in Figure 2. This constant magnetic field coil is of high strength, typically of the order of one or more Tesla.

The magnetic gradient coil 204 is configured to generate a spatially varying magnetic field in the volume of the bore, i.e. within a subject positioned on the patient support surface 114. The spatially varying magnetic field is in the same direction as the constant magnetic field produced by the primary magnetic field coil 202, i.e. in the z-direction. However, this magnetic field in the z-direction may vary (in strength) in dependence on location along x, y or z axes. The magnetic gradient coil 204 may include an x-direction magnetic gradient coil and/or a y-direction magnetic gradient coil and/or a z-direction magnetic gradient coil. Each of these gradient coils may be configured to generate a spatially varying field in the z-direction which varies along a particular axis. For example, the x-direction magnetic gradient coil may generate a magnetic field in the z-direction which varies along the x-axis. Similarly, the y-direction magnetic gradient coil may generate a magnetic field in the z-direction which varies along the y-axis. The z-direction magnetic gradient coil may generate a magnetic field in the z-direction which varies along the z-axis.

The RF coil 206 is configured to generate RF pulses and to detect MR signals produced in the subject in response to these RF pulses. When a current is passed through the RF coil 206, an oscillating/rotating magnetic field is produced in the bore. This magnetic field is perpendicular to the constant magnetic field produced by the primary magnetic field coil 202. Conversely, changing magnetic fields of the protons of the subject induce a voltage in the RF coil 206 which can be analysed to derive information on the distribution of tissue within the subject. The RF coil 206 may comprise a transmitter coil configured to generate the RF pulses and a receiver coil configured to detect the MR signals. Alternatively, the same coil may be used for both transmitting and receiving.

The MR imaging apparatus 112 may additionally comprise one or more of thermal insulation, magnetic shielding and RF shielding. These may be disposed between and/or around the outside of one or more of the coils described above. The MR imaging apparatus 112 may comprise an MR controller which controls the MR imaging apparatus 112. The MR controller may be a computer, processor, or other processing apparatus. The MR controller may be communicatively coupled to a memory, e.g. a computer readable medium. The MR controller may be communicatively coupled to one or more other components of the device 100 and/or to one or more controllers thereof.

In operation, MR images are generated using a pulse sequence, which may be stored in or communicated to the MR controller. The pulse sequence includes gradient pulses to be applied by the magnetic gradient coil 204. The pulse sequence also includes RF pulses to be applied by the RF coil 206. Furthermore, the pulse sequence may include signal acquisition operations, which may be applied using an analog-to-digital converter. The pulse sequence may define properties of the gradient pulses and/or of the RF pulses. These properties may include one or more of amplitude, timing, frequency, phase and duration.

In MR imaging, the response of hydrogen nuclei in water molecules within the subject to the pulse sequence can be used to generate images of the subject. A hydrogen nucleus, i.e. a proton, may be described as a rotating positive charge, the rotation of which generates a magnetic field. This magnetic field is referred to as a magnetic moment. The magnetic moments can only occupy one of two quantum states, known as spin-up or spin-down. When no magnetic field is applied magnetic moments are randomly orientated due to non-uniform thermodynamic fluctuations. The constant magnetic field generated by the primary magnetic field coil 202 acts to align and anti-align the magnetic moments in the subject with this constant magnetic field. Overall, a net magnetization can be generated as there is typically an excess of magnetic moments aligned (i.e. spin-up) than anti-aligned (i.e. spin-down) with the magnetic field. Alignment and anti-alignment is possible due to the interaction of the constant magnetic field with each proton, which creates a torque that causes it to precess around the direction of the constant magnetic field, i.e. the z-direction. The frequency $\omega_0$ of this precession is equal to the constant magnetic field strength $B_0$ multiplied by a constant $\gamma$ known as the gyromagnetic ratio. This gyromagnetic ratio has different known values depending on the nucleus being considered. For Hydrogen nuclei, the gyromagnetic ratio is $2.67 \times 10^8$ rad s$^{-1}$ T$^{-1}$. The Larmor frequency $\omega_0$ of precession of Hydrogen nuclei in the subject can therefore be calculated for a particular applied constant magnetic field $B_0$. Applying an RF pulse at the Larmor frequency tips the net magnetization of the hydrogen nuclei through a flip-angle $\alpha°$ (into the transverse plane). Immediately after the RF pulse is completed, the tipped net magnetization realigns with the constant magnetic field. This produces an MR signal at the Larmor frequency, which induces a measurable voltage in the RF coil 206.

The above mechanism provides means for detecting the quantity of hydrogen nuclei (protons) in the sample, and therefore for determining information about the composition of the sample. However, it does not discriminate between protons in one part of a subject and protons in another part of the subject. In other words, it provides means for determining a measure of the total number of hydrogen nuclei in the subject, but not for providing an image mapping these spatially.

In order to discriminate between nuclei located at different locations, the magnetic gradient coil 204 is used to vary the magnetic field spatially. For example, the z-direction magnetic gradient coil may generate a magnetic field that increases from a negative value at a first end of the bore to zero in the centre of the bore to a positive value at a second, opposite, end of the bore. The total field experienced by the nuclei is then the sum of the constant magnetic field $B_0$ and this spatially varying magnetic field. An RF pulse applied by the RF coil 206 may have a certain narrow bandwidth around the Larmor frequency. The nuclei at the centre of the bore will experience a field causing them to precess at this same Larmor frequency, since the spatially varying field is zero at that point, and resonance will therefore occur causing a measurable MR signal to be produced. Towards the first or second end of the bore, the magnetic field, and therefore the frequency of precession, will be smaller or larger respectively, meaning that resonance with the applied RF pulse will not occur and a measurable signal will not be produced from these regions. In effect, a central region of the bore (in the z-direction) has been interrogated by the applied signals. Therefore, applying a magnetic field gradient in a given direction provides means for selecting a particular spatial slice along that direction for measurement. For the magnetic field varying in the z-direction as described above, this spatial slice will be in the x-y plane and will have a slice thickness in the z-direction. Varying the carrier frequency of the applied RF pulse in the presence of the spatially varying magnetic field enables different slices along this direction to be measured. Applying gradients in a different direction would enable spatially selective measurements to be taken along those respective directions.

While application of a gradient enables a particular slice of the subject to be selected for measurement, this alone does not provide measurements discriminating between different locations in the selected slice. Such 'in-plane' measurements can be performed using phase-encoding and frequency encoding. In the above example, these in-plane measurements are in the x-y plane.

Phase encoding can be applied in a direction perpendicular to the applied gradient, e.g. in the x-direction. A phase-encode gradient can be applied by varying the magnetic field along the x-direction. This causes the precession of the nuclei to increase or decrease in frequency depending on their location along the x-axis (i.e. from the left to the right of the bore as shown in Figure 2). The spins of the nuclei are thereby caused to dephase to different extents. When the phase-encode gradient is turned off, the protons return to their original precession frequency, but retain this dephasing. However, protons at certain intervals along the x-direction will remain in phase due to the dephasing at these intervals being 360°, meaning that the protons at these intervals effectively remain in phase. The signals from these protons will add together rather than cancelling out and will therefore be measurable. Thus, applying a particular phase-encode gradient along the x-direction can be used to measure a sample at a particular interval or spatial frequency along the x-direction. The protons at that interval are picked out for measurement by the filter or comb that the application of the phase-encode gradient results in. Applying the RF signal multiple times, each time followed by application of a different respective phase-encode gradient, leads to a different filter or comb being applied since protons at different intervals will be picked out through being 360° out of phase (i.e. in phase). This enables different spatial locations in the x-direction to be measured.

To obtain measurements in the second in-plane direction, i.e. in the y-direction, frequency encoding can be used. A gradient is applied in the y-direction such that there is a slightly lower magnetic field at low y-values (towards the bottom of the bore as shown in Figure 2) and a slightly higher magnetic field at high y-values (towards the top of the bore as shown in Figure 2). This gradient is applied while the MR signal data is being measured. The gradient leads to slightly lower precession frequencies at the low y-values and slightly higher precession frequencies at the high y-values. The measured MR signal will comprise both the lower frequencies and the higher frequencies, as well as the frequency components between these extremes. The different frequencies in the MR signal can be differentiated and mapped to the different spatial locations along the y-direction.

Accordingly, the combination of phase encoding and frequency encoding provides in-plane measurements in the slice selected by the applied gradient. Varying the carrier frequency of the applied RF pulse enables different slices in a direction perpendicular to the plane of the slice to be measured. Therefore, these techniques enable three-dimensional information regarding a sample to be generated.

The magnetic fields of the nuclei induce a voltage in the radiofrequency coil 206, providing an MR signal. This output of the MR imaging process comprises raw data describing the frequencies of the MR signal. This raw data is k-space data. K-space data describes the spatial frequencies obtained from measurements of the sample. The k-space data can be represented as a 2D matrix of pixels with a frequency-encode axis and a phase-encode axis. Individual pixels within this matrix do not correspond to individual spatial locations of the subject. Instead, each pixel in the k-space matrix includes (partial) information relating to every spatial point that is measured in the sample. In order to obtain a spatial image from the k-space matrix, an inverse 2D Fourier transform can be applied to 'reconstruct' the image from the k-space data. This maps the measurements obtained, which are in terms of spatial frequencies, to measurements in terms of spatial locations within the sample. Where 3D k-space data is obtained and considered, the k-space matrix may be a 3D matrix of pixels with an additional dimension corresponding to the magnetic gradient direction. An inverse 3D Fourier transform may be applied to reconstruct a 3D image (i.e. 3D spatial information) from the k-space data.

While the above explanation has focused on a Cartesian sampling scheme in which the measurements are taken along a set of orthogonal axes, non-Cartesian sampling schemes are also possible. In non-Cartesian sampling schemes, measurements may be taken along radial or spiral trajectories, in which k-space data are obtained along overlapping 'spokes'. For example, 'stack-of-stars' sampling may be applied, which corresponds to a cylindrical coordinate system comprising radial spokes in-plane (e.g. in the x-y plane) and Cartesian sampling perpendicular to this plane (e.g. in the z-direction). In such radial sampling schemes, frequency encoding may be applied simultaneously in two directions (e.g. the x and y directions), rather than applying frequency encoding in one of these directions and phase encoding in the other of these directions.

Different types of tissue within the sample have different quantities of different atoms, which each have different proton densities and different spin-spin relaxation times and spin-lattice relaxation times. For example, fluids such as blood and spinal fluid have a higher proton density than bone due to containing a larger proportion of hydrogen atoms. As explained above, the RF signal applied can be tailored to cause a resonant response in hydrogen nuclei. The differences in the compositions of different body tissues enable these different types of tissue to be discriminated in the MR signal received. This enables generation of an image which shows different types of tissue with different contrast. For example, an image can be reconstructed which shows unhealthy tissue, such as a tumour, either lighter or darker than surrounding healthy tissue.

Generating an image depicting the prevalence of different types of tissue within a subject can be used to inform and/or guide treatments applied to the subject. For example, such an image may be used to guide radiotherapy treatment. The image may be used in treatment setup to determine a treatment plan, i.e. to determine an arrangement of angles and/or locations at which to apply radiotherapy. Alternatively, or in addition, the image may be generated and/or used during radiotherapy treatment to determine whether to halt or pause treatment. However, measuring, reconstructing and analysing 2D k-space data is a time-intensive task. If a subject moves such that the location of the application of radiation and the location of the unhealthy tissue no longer coincide, the unhealthy tissue may receive a lower radiation dose than is desired and healthy tissue may receive a higher radiation dose than is desired. A beam may be gated in response to determining this movement based on the reconstructed image. However, the latency of such gating may be greater than desired. The unhealthy tissue may receive the lower than desired dose, and the healthy tissue may receive the higher than desired dose, for a length of time corresponding to the gating latency.

Previous approaches have focused on using the raw k-space data generated in MR imaging as a stepping stone to deriving reconstructed images of the anatomy of a subject. Deriving images from MR imaging data is the conventional way of using this diagnostic. Since a tumour in a subject and a dose delivered by a radiotherapy beam are described in terms of their spatial locations, the reconstructed images provide means for relating these locations to each other at a given time. The reconstructed images may also be more readily understood by an operator since they provide information in the form of spatial locations of different tissues, which corresponds with their everyday experience of how anatomy is thought of and viewed. Previous techniques have therefore focused on reconstructing images of a subject, or shifting images of a subject based on movement of a subject, and then using such images as the basis for taking any action with respect to treatment. However, it has been found according to the present disclosure that a radiotherapy beam can be gated based more directly on the central k-space data itself. The gating can be based on non-spatial domain data. The gating can be based on central k-space data, rather than based on images reconstructed from k-space data. This reduces the latency of gating and thereby enables improved safety, more efficient treatment and improved clinical outcomes to be achieved.

Data in the centre of a k-space matrix (i.e. corresponding to low spatial frequencies) contain contrast and signal to noise information for the entire spatial image. Data at the edges of the k-space matrix (i.e. corresponding to high spatial frequencies) contain information describing the edges and boundaries for the entire spatial image. Central k-space data may be obtained by applying a pulse sequence as described above. The k-space data may be filtered to remove data from the edges of k-space. In other words, the central k-space data may be selected or filtered from the k-space data. The central k-space data may comprise a single data point at the centre of k-space. The central k-space data may comprise a number of data points at and around the centre of k-space. The number of data points selected may be less than the total number of data points obtained. In a radial or spherical geometry centred at zero and extending to a maximum extent R which corresponds to the total k-space data obtained, the data points at 0, 1, 2, ..., r may be selected, wherein r < R. The integer r may be 1, 2, 5, 10, 20, or any other suitable value.

Reconstruction of images when non-Cartesian sampling, such as radial sampling, is used may take even longer than reconstruction of images when Cartesian sampling is used since the data points acquired are 'gridded' into a rectangular matrix before they are Fourier transformed.

The techniques of the present disclosure may be performed using a pulse sequence which repetitively samples the centre of k-space. For example, in the radial stack-of-stars trajectory, an in-plane spoke (e.g. in the x-y plane) is acquired at the same angular increment for all phase encoding steps along the slice dimension. This acquisition pattern is repeated for a chosen number of different angular increments. This means that one spoke for each angular increment provides a measurement of the centre of k-space, increasing the frequency with which the centre of k-space is measured. This increased frequency with which the centre of k-space is measured can further reduce the latency of gating because each determination of whether to gate the radiotherapy beam can be based on each new measurement of central k-space data. The centre of k-space may be sampled, for example, at approximately 10 Hz. In some examples, the stack-of-stars trajectory will be combined with golden-angle or tiny golden-angle sampling. Moreover, in some examples, the ROtating Cartesian K-space sequence may be used.

The central k-space data can be described in terms of its magnitude, phase, real component and imaginary component. For a single data point at the centre of k-space ($k_x$, $k_y$, $k_z$ = 0), its real and imaginary parts may be summed to give a centre of mass respiratory surrogate signal. Additionally, for the stack-of-stars sequence, a centre of mass self-gating signal can be obtained from projection data. Projection data may be computed by applying a one-dimensional inverse fast Fourier transform along the slice-dimension on the k-space data passing through, or close to, the axis of rotation ($k_x$, $k_y$ = 0). From the projection of the k-space centre data ($k_x$, $k_y$ = 0), a self-gating signal may be obtained using principal component analysis. The first principal component calculated corresponds to the axis of largest movement of the subject. Alternatively, a self-gating signal may be determined by combining the projection data obtained from several points around and including the k-space centre, for instance by averaging. Calculated self-gating signals may be band-pass filtered to reduce the influence of frequencies outside the range of respiratory motion, such as those associated with the cardiac cycle. Self-gating signals calculated from separate receive coils can be combined using averaging or principal component analysis. Alternatively, self-gating signals from different receive coil elements may be combined using coil clustering techniques based on cross-correlation, eigenvalue decomposition and thresholding.

When a subject or part of the subject moves during a radiotherapy treatment, this will be manifested in the central k-space data. By way of example, respiratory motion of the subject leads to movement of the diaphragm of the subject along the superior-inferior axis, i.e. along the z-axis of the MR imaging apparatus 112. Treatment sites connected to the diaphragm, such as the liver or pancreas, move in a corresponding manner. The self-gating signal described above correlates with this movement along the z-axis. Therefore, for targets (i.e. tumours) in the liver or pancreas, this exemplary self-gating signal can be used to determine when to apply a radiotherapy beam and when to gate the radiotherapy beam.

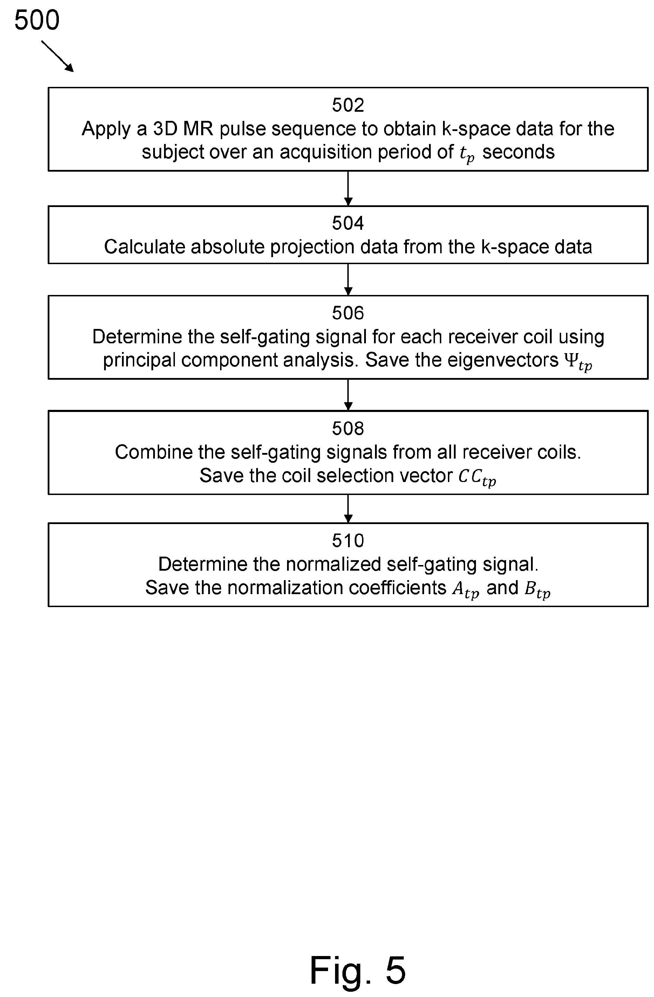

The self-gating signal can be extracted using the following method, which is described in Figure 5 500. First, using a 3D stack-of-stars pulse sequence, a training set of complex k-space data $K(k_x, k_y, k_z, t, c)$ may be acquired 502, where $k_x$, $k_y$, $k_z$ represent the $x$, $y$, $z$ vector components of k-space, respectively. Additionally, $t$ represents the temporal component, which has a range between 0 and the training time $t_p$. Finally, $c$ is used to express the receiver coil dimension, which lies between 1 and $N_c$ ($N_c$ denoting the number of coil elements). The training time period $t_p$ may be greater than or equal to the period of the subject's respiratory waveform. Next, absolute three-dimensional projection data $P(z, t, c)$ may be calculated by applying a one-dimensional inverse fast Fourier transform, along the slice-dimension, to the acquired central k-space matrix 504:

$$P(z,t,c) = \left| f^{-1}\big(K(0,0,k_z,t,c)\big)\right|$$

Afterwards, the projection data $P(z, t, c)$ may be normalized. Normalization is performed separately for each temporal and coil element:

$$P_n(z,t,c) = \frac{P(z,t,c) - Argmin_z\big(P(z,t,c)\big)}{Argmax_z\big(P(z,t,c)\big) - Argmin_z\big(P(z,t,c)\big)}$$

Separately for each coil element, principal component analysis may then be applied to the normalized projection data 506. First, $P_n(z, t)$ may be reformatted to permute rows and columns: $P_n(t, z)$. Subsequently, $P_n(t, z)$ may be centred. In this operation, the mean row is calculated and then separately deducted from each row in $P_n(t,z)$:

$$P_m(t,z) = P_n(t,z) - \frac{1}{Nt_p}\sum_{n=1}^{Nt_p} P_n(t,z)$$

Note that $N_{tp}$ denotes the total number of rows in $P_n(t, z)$. Based on the centred projection data $P_m(t,z)$, the covariance matrix $C(z, z)$ may next be calculated:

$$C(z,z) = \frac{1}{Nt_p - 1} P_m^T(t,z)P_m(t,z)$$

The diagonalized eigenvalues $\lambda(z, z)$ and eigenvectors $\Psi(z, z)$ of the covariance matrix $C(z, z)$ may then be obtained by eigendecomposition:

$$C(z,z)\Psi(z,z) = \Psi(z,z)\lambda(z,z)$$

The eigenvectors obtained using the centred projection data $P_m(t, z)$, which were acquired over a time period of $t_p$ seconds, should be saved for later use:

$$\Psi_{t_p}(z,z) = \Psi(z,z)$$

The eigenvectors $\Psi(z, z)$ represent an orthogonal linear transformation, which may be applied to transform $P_m(t, z)$ to a new domain:

$$T(t,z) = P_m(t,z)\Psi(z,z)$$

In the new domain, the highest variance in the projection data $P_m(t, z)$ is associated with the eigenvector with the largest corresponding eigenvalue (i.e. the first principal component). This is typically the respiratory self-gating signal:

$$S(t) = T(t,1)$$

Band-pass filters may be applied to the self-gating signal to reduce the influence of noise and undesirable frequencies.

The above-described procedure may be repeated to obtain multiple self-gating signals for different coil elements:

$$S(t, c) = T(t, 1, c)$$

The self-gating signals $S(t, c)$ obtained from various coils may be combined into a single signal $S(t)$ by averaging or principal component analysis 608. Alternatively, signals may be combined using coil clustering techniques. In some examples of clustering methods, signals are combined by selecting and then averaging the motion estimates from a subset of coils from the $N_c$ coil elements. Coil selection may be performed based on cross-correlation, eigendecomposition and thresholding of the measured signals $S(t, c)$. The selection of signals $CC_{tp}$ combined from $S(t, c)$, corresponding to data acquired over a time period of $t_p$ seconds, should be saved for later use.

Finally, the self-gating signal $S(t)$ may be normalized 610, which facilitates further signal analysis:

$$A = Argmin_t\big(S(t)\big) ; B = Argmax_t\big(S(t)\big) - Argmin_t\big(S(t)\big)$$

$$S_n(t) = \frac{S(t) - A}{B}$$

The normalization coefficients $A_{tp} = A$; $B_{tp} = B$, corresponding to data acquired over a time period of $t_p$ seconds, should be saved for later use.

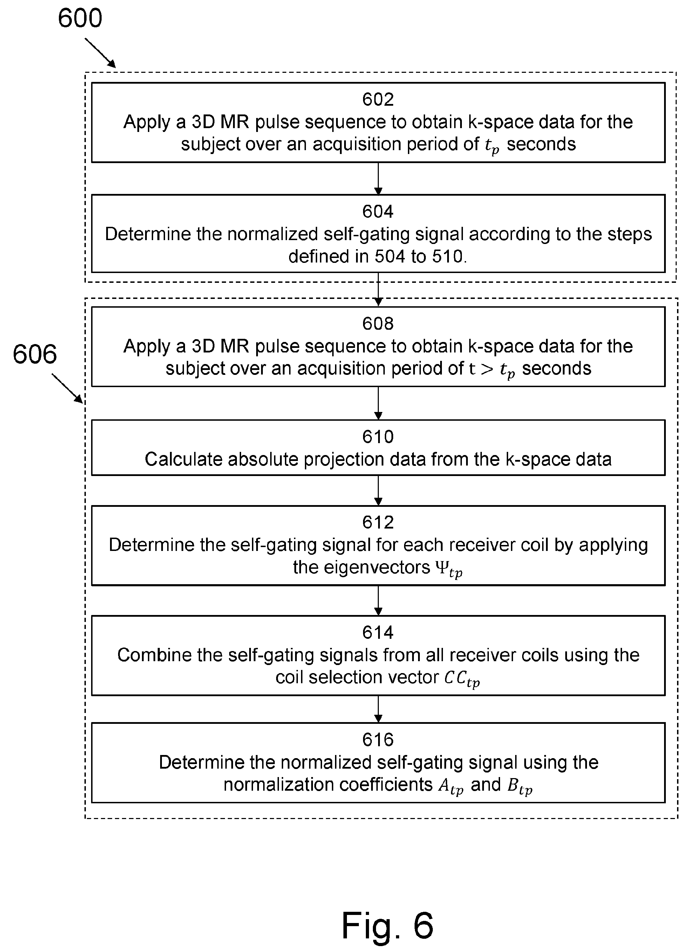

When calculating the self-gating signal at time-points $t > t_p$, a variation of the above-described method may be applied to obtain $S_n(t)$ (Figure 6, method 600). The following method assumes that $S(t)$ has already been acquired and processed up to the training time $t_p$ (602 and 604). First, the centred projection data $P_m(\Delta t, z)$, corresponding to a new acquisition time-point $\Delta t$, may be acquired 608 and processed 610 following the same techniques as previously described. For each coil element, the projection data $P_m(t, z)$, where $t$ is in the range $t_p + \Delta t$, may then be obtained by concatenating (denoted as: ‖) the newly processed projection data with pre-calculated projection data:

$$P_m(t, z) = P_m\big(t_p, z\big) || P_m(\Delta t, z)$$

For subsequent acquisition time-points, the above-described method is repeated except replacing $P_m(t_p, z)$ with the pre-calculated projection data from the previous acquisition time-point:

$$P_m(t, z) = P_m(t, z) || P_m(\Delta t, z)$$

$$t = t + \Delta t$$

Afterwards, $P_m(t, z)$ may be transformed to the new domain using the previously calculated eigenvectors 612:

$$T(t, z) = P_m(t, z)\Psi_{tp}(z, z)$$

Applying the same transformation $\Psi_{tp}(z, z)$ for time-points $t > t_p$ facilitates consistent scaling of $T(t, z)$. Afterwards, the self-gating signal may be calculated from $T(t, z)$ in the same approach as previously described:

$$S(t, c) = T(t, 1, c)$$

The self-gating signal $S(t)$ can be found from $S(t, c)$ for instance by coil clustering 614. To enforce consistent scaling of $S(t)$, clustering may be performed using the previously calculated coil selection $CC_{tp}$.

Finally, the self-gating signal should be normalized 616. To keep the scaling consistent between time-points, the previously calculated normalization coefficients may be utilised:

$$S_n(t) = \frac{S(t) - A_{tp}}{B_{tp}}$$

The steps 608-616 may be referred to collectively as step 606. The above-described approach to obtain $S_n(t)$ reduces the required computation time by obviating redundant processing steps. In particular, the previously calculated projection data $\boldsymbol{P_m}$, eigenvector matrix $\Psi_{Tp}$, coil selection vector $CC_{tp}$ and normalization coefficients $A_{tp}$ and $B_{tp}$ may be reused.

The above-described techniques provide consistent scaling of the self-gating signal. In other words, the intensity of the self-gating signal is consistent between different time points. In determining the self-gating signal for a second time point subsequent to a first time point, the whole signal may be reanalysed using principal component analysis. For the second, and later, time points, the calculated amplitude of the self-gating signal may be different to the amplitude of the self-gating signal calculated for the first time point. As well as expected changes in amplitude between time-points due to respiratory motion, undesirable changes in amplitude between time-points due to jitter may also be present. This jitter in the self-gating signal involves its amplitude varying suddenly and/or irregularly, rather than describing smooth oscillation. This jitter can lead to incorrect gating decisions being made due to the jitter causing the self-gating signal to fall within or outside of the gating window. In contrast, the above-described techniques involve the reuse of parameters between timepoints, which reduces the jitter and provides more accurate gating.

Therefore, the reuse of parameters according to the techniques described above enables consistent scaling of the self-gating signal between time points and reduces gating latency by avoiding recalculation of such parameters for each time point. In addition, since the bulk of the processing is applied to 2D matrices (i.e. projections x coils), rather than to 3D matrices (i.e. readout x phase encoding x coils), the calculations may be less computationally intensive according to the method described above and therefore the time taken to perform the calculations may be reduced. This may further reduce the gating latency. The described techniques enable the self-gating signal to be calculated more quickly. The self-gating signal can be calculated in near real-time rather than retrospectively based on previously acquired (e.g. pre-treatment) MR imaging data. The beam gating control signal can be generated during a radiotherapy treatment based on data acquired during the radiotherapy treatment.

The self-gating signal $S_n(t)$ can be used to drive gating according to the following technique. First, $S_n(t)$ may be processed to define a gating window $G_{win}(x, t)$:

$$G_{win}(x,t) = A_{win}(x,\alpha,\beta) \cdot \left[ Argmax_t\big(S_n(t - t_a : t)\big) - Argmin_t\big(S_n(t - t_a : t)\big) \right]$$
$$+ Argmin_t\big(S_n(t - t_a : t)\big)$$

$$A_{win}(x,\alpha,\beta) = \Theta(x - \alpha) - \Theta(x - \beta)$$

Where $A_{win}(x, \alpha, \beta)$ denotes a boxcar function controlling the gating window extent. Note that $x$ represents the gating window space: $0 \le x \le 1$. Furthermore, $\alpha$ and $\beta$ are constants: $0 \le \alpha \le 1$ and $0 \le \beta \le 1$. The gating window extent may be dynamically updated throughout treatment delivery using $S_n(t)$ in the temporal range $t - t_a$ to $t$, where $t_a$ is a temporal offset value. The temporal offset value $t_a$ should be less than or equal to the training time period $t_p$, otherwise there will be insufficient data to support definition of the gating window.

Gating may be controlled based on the amplitude value of $S_n(t - t_a : t)$:

$G_{win}(a, t) < S_n(t - t_a : t) < G_{win}(\beta, t)$ : Beam-on: structure is inside the gating window

$S_n(t - t_a : t) \le G_{win}(\alpha, t)$ or $S_n(t - t_a : t) \ge G_{win}(\beta, t)$: Beam-off: structure is outside the gating window

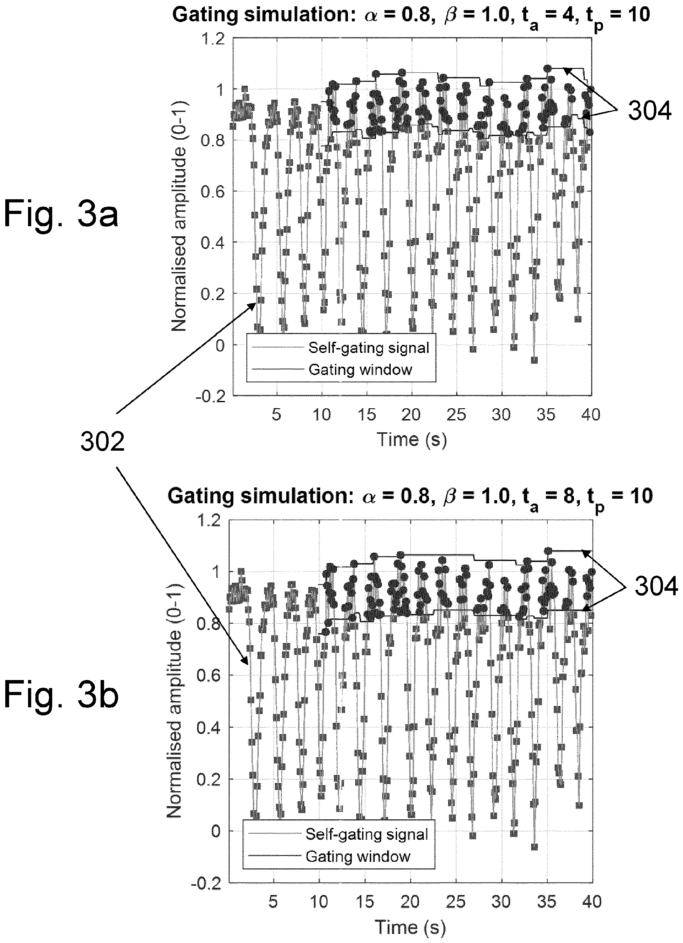

Figures 3a and 3b depict example dynamic gating window simulations for an exhalation-based delivery. These simulations use values of $\alpha$ =0.8, $\beta$ =1.0, $t_p$ = 10 s, with Figure 3a using temporal offset value $t_a$ =4.0, and Figure 3b using temporal offset value $t_a$=8.0 s. In other examples of the present disclosure, other parameters may be used. The self-gating signal 302 is shown oscillating between the top and the bottom of Figure 3a as the subject moves. The gating window 304, between the approximately horizontal lines towards the top of Figure 3a, indicates the region at which the radiotherapy beam should be applied. The upper approximately horizontal line indicates the maximum value of the self-gating signal at which radiotherapy should be applied. The lower approximately horizontal line indicates the minimum value of the self-gating signal at which radiotherapy should be applied. When the self-gating signal 302 moves outside this gating window 304 (e.g. moves below it), the beam is gated. As shown in Figure 3a and Figure 3b, increasing the magnitude of the temporal offset value $t_a$ from 4 to 8 seconds increases the temporal stability of the gating window with respect to sudden changes in respiratory motion. A longer temporal offset may be advantageous for treatments focussed on accounting for baseline drift, such as tumour trailing deliveries at midposition. A tuneable temporal offset parameter enables the stability of the gating window to be optimized for the unique baseline drift exhibited by each patient's respiratory waveform.

The controller of the radiotherapy device 100, or the MR controller, may continuously, at intervals, or at a series of predetermined times, determine whether the self-gating signal 302 is within the gating window 304. If the self-gating signal 302 moves outside the gating window 304, the controller may generate a beam gating control signal. The controller may transmit this beam gating control signal to the radiation source, which may gate the radiotherapy beam in response to the beam gating control signal. If the self-gating signal 302 moves inside the gating window 304, the controller may generate a beam-on control signal. The controller may transmit this beam-on control signal to the radiation source, which may turn the radiotherapy beam on (or back on) in response to the beam-on control signal.

The present disclosure is applicable to various different treatment options, including mid-ventilation delivery (geometric mean position of the respiratory cycle), midposition delivery (time-weighted mean position of the respiratory cycle), inhalation-based delivery, exhalation-based delivery and tumour-trailing delivery.

The above non-limiting example is provided for ease of understanding in order to explain the principles involved. Other parts of a subject may move in different ways, which may be manifested in the central k-space data in corresponding manners. For example, sudden movements of the subject such as due to coughing or sneezing will correspond to further, different motions of the subject or parts thereof. Since the diaphragm moves during such movements, they may manifest in the k-space data in a similar manner to that described above. For such movement, the associated self-gating signal will not show the same cyclical variation as that associated with the respiratory cycle of the subject. However, it may manifest as a sudden deviation which moves the self-gating signal outside of the gating window depicted in Figures 3a and 3b therefore may lead to the radiotherapy beam being gated in response. In another example, for targets close to the heart, cardiac motion may be considered. This is possible because, in addition to respiratory motion information, the self-gating signal contains cardiac motion information. The self-gating cardiac signal can be similarly obtained from k-space data as described above. However, for the cardiac case, frequencies corresponding to the respiratory cycle might instead be bandpass filtered in order to reduce their influence on the derived self-gating signal. Moreover, the principal component corresponding to cardiac motion is instead extracted.

Gating the radiotherapy beam enables selectively applying radiation in some time periods and not in others. The radiation source may comprise a particle source, for example an electron source 106 and an RF field source 102 (as shown in Figure 1). The electron source may provide a source of electrons which generate a radiation dose to be delivered to the subject, for example by impacting a target. The RF field source may electromagnetically accelerate the electrons to a desired velocity suitable for providing the radiation dose. The radiation source may be gated by selectively controlling the electron source to be on (active) or off (inactive). Alternatively, or in addition, the radiation source 100 may be gated by selectively controlling the RF field source to be on (active) or off (inactive). In this manner, application of a radiation dose by the radiation source can be controlled according to desired parameters, for example based on a beam gating control signal (and a beam-on control signal) according to the present disclosure.

The radiation source may comprise a radiation source controller suitable for controlling the radiation source, for example by gating the radiation source, stopping gating of the radiation source and/or adjusting a dose rate of the radiation source. The controller may transmit the control signal to the radiation source controller. The control signal may comprise instructions for effecting such gating or stopping such gating or adjusting the dose rate immediately, at a defined later time, after a defined interval, or some combination of these. The control signal may therefore comprise instructions for time-varying or time-dependent gating of the beam by the radiation source and/or adjustment of the dose rate by the radiation source.

As described above, according to previous approaches, an image of a subject may be reconstructed and used to determine motion of a part of the subject, which may in turn be used to determine whether to gate a radiotherapy beam. The presently described techniques instead drive gating of a radiotherapy beam based on central k-space data, i.e. without being based on a reconstructed image. A controller may generate a beam-gating control signal without and/or independently of and/or before reconstructing an image based on k-space data. The controller may generate the beam-gating control signal without a causal link between reconstructing an image and generating the beam-gating control signal. The controller may generate a beam-gating control signal based directly on the central k-space data. The controller may generate a beam-gating control signal without use of non-central k-space data, i.e. without use of k-space data which is not at the centre of k-space. The controller may generate a beam-gating control signal from or using the central k-space data. This is beneficial for reducing gating latency.

While, according to the present disclosure, the gating of the radiotherapy beam is driven based on the central k-space data, rather than based on reconstructed MR images, MR images may still be reconstructed (during and/or after the treatment) from the k-space data that is obtained. The controller may be configured, after generating the beam gating control signal, to reconstruct an image of the subject based on the acquired k-space data and processed self-gating signal. The processing of the self-gating signal may be used to sort the acquired k-space data into different respiratory phases of the subject. As more and more k-space data are obtained, MR images can be iteratively updated to include the additional k-space data. As additional spokes through the k-space data are obtained, the signal to noise ratio of the k-space data is increased and the detail of the reconstructed MR images is enhanced. The spoke acquisition frequency is approximately equivalent to the repetition time of the pulse sequence (which may, for example, be approximately 3-8 ms for the stack-of-stars sequence). If the Nyquist criterion is not met (i.e. too few spokes are acquired), undersampling artefacts will manifest in the images as noisy streaks.

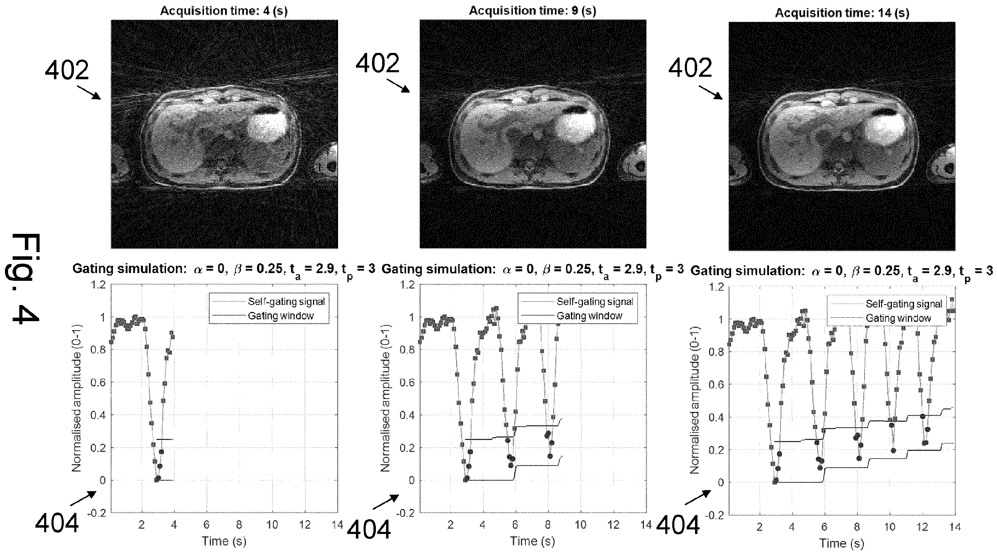

Figure 4 depicts an example of this iterative image reconstruction. The left-hand panels of Figure 4 depict time-averaged stack-of-stars images 402 acquired with different numbers of spokes increasing from bottom to top. When fewer spokes are acquired, the Nyquist criterion is not yet met and heavy streaking artefacts are exhibited. The right-hand panels of Figure 4 depict plots of gating parameters 404. The respective right-hand panels correspond to the respective left-hand panels. Therefore, the right-hand panels depict the gating parameters at later and later times when viewed from bottom to top. More of the signal is visible in the plot moving from bottom to top. The normalised self-gating signal is shown as an oscillating line. The approximately horizontal lines indicate the upper and lower extents of the gating window for the parameters: : $\alpha$ =0, $\beta$ =0.25, $t_p$=3s, $t_a$ =2.9s. The points of the self-gating signal within the gating window represent times when a gating criteria is fulfilled, when a beam-on control signal may be generated to turn on the radiotherapy beam.

Therefore, while the presently described techniques enable gating of a radiotherapy beam based on central k-space data rather than reconstructed images, reconstruction of such images can still be performed. This reconstruction may be performed using one or more algorithms known in the art, such as the GRASP 4D algorithm or the joint MoCo-HDTV algorithm. When combined with synthetic-CT (computed tomography), retrospectively reconstructed MR image data can be used for offline dose reconstruction. In offline dose reconstruction, the true dose given to the patient is estimated by re-calculating the prescribed treatment plan on the reconstructed image acquired during treatment delivery. The estimate of the delivered dose is then utilised to optimise the dose prescribed in subsequent treatment deliveries. Since 3D k-space data is obtained, the techniques of the present disclosure do not suffer from the limited field-of-view or through-plane gradient non-linearity geometrical distortion associated with using 2D techniques, which can limit the accuracy of dose reconstruction. Moreover, sequence-dependent distortions originating from susceptibility artefacts, static field inhomogeneities and chemical shift have a reduced impact on images acquired using non-Cartesian radial sequences, such as the 3D stack-of-stars sequence, compared to those obtained using 2D Cartesian sequences. This is because sequence-dependent distortions manifest in non-Cartesian radial images as signal blurring, due to the radial readout, rather than as systematic shifts along the readout direction as in images acquired using a 2D Cartesian sequence.

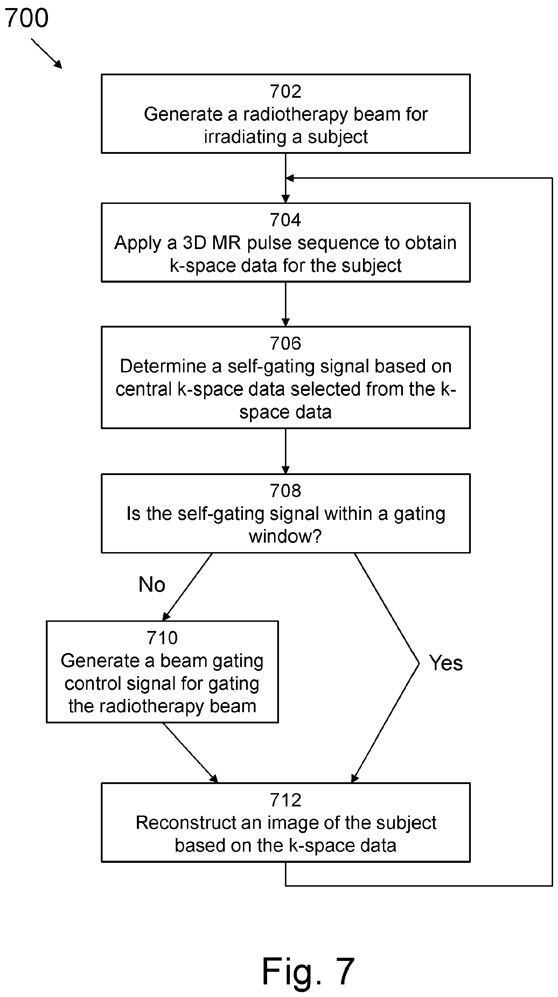

A method 700 for integrated MR imaging and radiotherapy delivery is depicted in Figure 7. This depicts integration of the gating and the dose reconstruction into a motion-monitoring workflow.

In a step 702, a radiotherapy beam for irradiating a subject may be generated. The radiotherapy beam may be generated by a radiation source of the radiotherapy device 100. The radiotherapy beam may irradiate a target region, for example a tumour, within the subject.

In a step 704, a 3D MR pulse sequence may be applied to obtain k-space data for the subject. In some examples, a 3D MR pulse sequence may be applied to obtain central k-space data for the subject. The 3D MR pulse sequence may be applied while the radiotherapy beam is applied, i.e. in real time, during treatment. The 3D MR pulse sequence may be applied by the MR imaging apparatus 112. The k-space data may be or comprise central k-space data. As used herein, a 3D MR pulse sequence refers to a pulse sequence which has a 3D k-space trajectory.

In a step 706, a self-gating signal may be determined based on central k-space data selected from the k-space data. This determination may be made using the calculations and/or processing techniques described above, for example in relation to Figures 5 and 6. The selection of the central k-space data may be performed by the MR imaging apparatus 112, a controller thereof, or a controller of the radiotherapy device 100. The self-gating signal may be determined by the controller of the radiotherapy device 100.

In a step 708, it may be determined whether the self-gating signal is within a gating window. The gating window may define the range of acceptable values for the self-gating signal for which treatment should be continued. The determination may be made by the controller of the radiotherapy device 100.

In a step 710, if the self-gating signal is not within the gating window, a beam gating control signal may be generated for gating the radiotherapy beam. The beam gating control signal may be generated based on the central k-space data and/or based on the self-gating signal. The beam gating control signal may be generated by the controller of the radiotherapy device 100. The controller may transmit the beam gating control signal to the radiation source. The radiation source may gate the radiotherapy beam based on or in response to the beam gating control signal. The method may then proceed to step 712. If the self-gating signal is within the gating window, the method may proceed directly from step 708 to step 712.

In the step 712, an image of the subject may be reconstructed based on the k-space data. The reconstruction may be performed by the controller of the radiotherapy device 100 or a controller of the MR imaging apparatus 112. The method may return to step 704 such that the remainder of the 3D MR pulse sequence or additional 3D MR pulse sequences are applied. For subsequent iterations of step 704 in which additional k-space data is obtained, subsequent iterations of step 712 may comprise updating the reconstructed image of the subject based on the additional k-space data. Each subsequent iteration during treatment makes the image clearer as the Nyquist criterion is reached. The reconstructed image may be used for dose reconstruction.

The method may return to step 704 while reconstruction of the image is being performed. In other words, one or more of obtaining additional k-space data, redetermining a self-gating signal, redetermining whether the self-gating signal is within the gating window, generating a beam gating control signal and gating a radiotherapy beam can be performed while reconstruction of the image is being performed. This reduces gating latency since these steps may be performed without waiting for image reconstruction or analysis of reconstructed images.

In subsequent iterations of the loop, if the radiotherapy beam has been gated, and if it is determined that the self-gating signal is within the gating window at step 708, a beam-on control signal may be generated. The beam-on control signal may be generated based on the central k-space data and/or based on the self-gating signal. The beam-on control signal may be generated by the controller of the radiotherapy device 100. The controller may transmit the beam-on control signal to the radiation source. The radiation source may turn (back) on the radiotherapy beam based on or in response to the beam-on control signal.

The step 712 may be performed for some iterations of the loop but not for others. For example, the step 712 may be performed for every $n^{th}$ iteration of the loop, where n is an integer. The integer n may be 1, 2, 5, 10, 20, or any other suitable value. The step 712 may be performed in response to a predefined time period having elapsed since it was last performed.

The image may be displayed on a display during and/or after treatment. Parameters of the treatment plan may be superimposed on the image. The self-gating signal and/or the gating window may be displayed in a gating interface, on the same or a separate display.

As will be appreciated from the method described above, the beam gating control signal is generated based on the central k-space data before the image of the subject is reconstructed. This can reduce the latency of gating. The image of the subject can be reconstructed during treatment and iteratively made more detailed as the treatment progresses. This provides faster and more accurate imaging of the subject.

While the methods disclosed herein are presented in a certain sequential order, this should not be taken to limit the methods to the orders presented. One or more of the method steps may be omitted or rearranged. The various steps may be performed in different orders. Various steps may be performed at the same time or substantially the same time. Herein, references to events occurring substantially at the same time may refer to events at least partially overlapping in time and/or events occurring at the same time within measurement uncertainties.

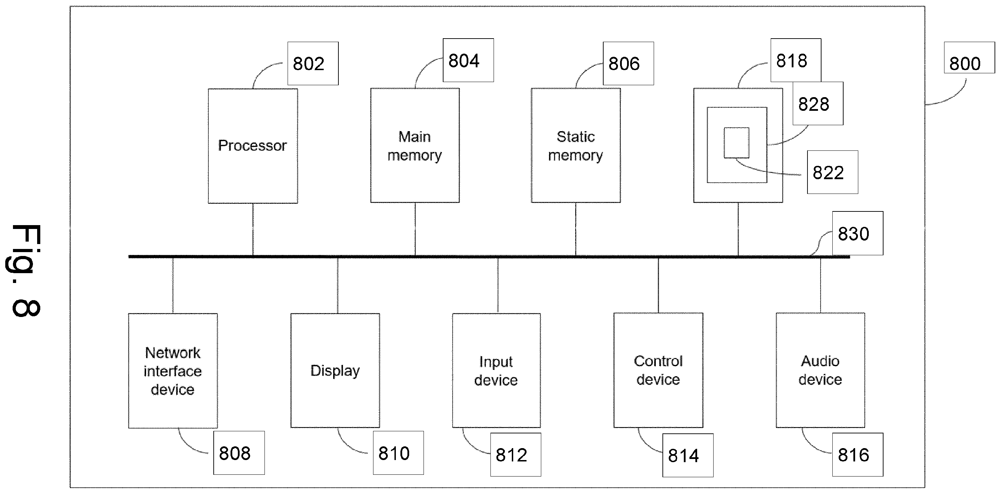

Figure 8 illustrates a block diagram of one implementation of a computing device 800 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 800 includes a processing device 802, a main memory 804 (e.g., read-only memory (ROM), flash memory, dynamic random access memory (DRAM) such as synchronous DRAM (SDRAM) or Rambus DRAM (RDRAM), etc.), a static memory 806 (e.g., flash memory, static random access memory (SRAM), etc.), and a secondary memory (e.g., a data storage device 818), which communicate with each other via a bus 830.

Processing device 802 represents one or more general-purpose processors such as a microprocessor, central processing unit, or the like. More particularly, the processing device 802 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 802 may also be one or more special-purpose processing devices such as an application specific integrated circuit (ASIC), a field programmable gate array (FPGA), a digital signal processor (DSP), network processor, or the like. Processing device 802 is configured to execute the processing logic (instructions 822) for performing the operations and steps discussed herein.

The computing device 800 may further include a network interface device 808. The computing device 800 also may include a video display unit 810 (e.g., a liquid crystal display (LCD) or a cathode ray tube (CRT)), an alphanumeric input device 812 (e.g., a keyboard or touchscreen), a cursor control device 814 (e.g., a mouse or touchscreen), and an audio device 816 (e.g., a speaker).

The data storage device 818 may include one or more machine-readable storage media (or more specifically one or more non-transitory computer-readable storage media) 828 on which is stored one or more sets of instructions 822 embodying any one or more of the methodologies or functions described herein. The instructions 822 may also reside, completely or at least partially, within the main memory 804 and/or within the processing device 802 during execution thereof by the computer system 800, the main memory 804 and the processing device 802 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying," "applying, " "transmitting," "generating," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The approaches described herein may be embodied on a computer-readable medium, which may be a non-transitory computer-readable medium. The computer-readable medium may carry computer-readable instructions arranged for execution upon a processor so as to cause the processor to carry out any or all of the methods described herein. The computer-readable medium may comprise computer-executable instructions for generating a control signal for a radiotherapy device. The computer-readable medium may comprise computer-executable instructions which, when executed by a processor of a radiotherapy device, cause the radiotherapy device to perform any of the methods described herein.

The term "computer-readable medium" as used herein refers to any medium that stores data and/or instructions for causing a processor to operate in a specific manner. Such storage medium may comprise non-volatile media and/or volatile media. Non-volatile media may include, for example, optical or magnetic disks. Volatile media may include dynamic memory. Exemplary forms of storage medium include, a floppy disk, a flexible disk, a hard disk, a solid state drive, a magnetic tape, or any other magnetic data storage medium, a CD-ROM, any other optical data storage medium, any physical medium with one or more patterns of holes, a RAM, a PROM, an EPROM, a FLASH-EPROM, NVRAM, and any other memory chip or cartridge.

The invention is defined in the following claims. Other embodiments, examples, methods, items etc. are not a part of the invention.

**Claims**

1. A radiotherapy device (100) comprising:

a radiation source configured to generate a radiotherapy beam (110) for irradiating a subject;
an MR imaging apparatus (112) configured to apply a 3D MR pulse sequence to obtain k-space data for the subject; and

a controller communicatively coupled to the radiation source and the MR imaging apparatus, wherein the controller is configured to:

select central k-space data from the k-space data;

generate a beam gating control signal based on the central k-space data; and,

after generating the beam gating control signal, reconstruct an image of the subject based at least in part on the k-space data.

2. A radiotherapy device (100) according to claim 1, wherein the controller is configured to generate the beam gating control signal independently of reconstructing an image of the subject based at least in part on the k-space data.

3. A radiotherapy device (100) according to claim 1 or claim 2, wherein the controller is configured to transmit the beam gating control signal to the radiation source, and the radiation source is configured to gate the radiotherapy beam (110) based on the beam gating control signal.

4. A radiotherapy device (100) according to any preceding claim, wherein the central k-space data comprises a single pixel at the centre of k-space, and/or wherein the central k-space data comprises a plurality of pixels at and surrounding the centre of k-space.

5. A radiotherapy device (100) according to any preceding claim, wherein the MR imaging apparatus (112) is configured to obtain additional k-space data, and wherein the controller is configured to update the reconstructed image of the subject based at least in part on the additional k-space data.

6. A radiotherapy device (100) according to any preceding claim, wherein the 3D MR pulse sequence is arranged to repetitively sample the centre of k-space as part of its k-space trajectory.

7. A radiotherapy device (100) according to any preceding claim, wherein the 3D MR pulse sequence comprises a radial stack-of-stars sampling trajectory in which the 3D MR pulse sequence is arranged to repetitively sample the centre of k-space by sampling along a plurality of spokes, each of the spokes being rotated by an angular increment relative to a previous spoke, and/or comprises a stack-of-spirals sampling trajectory, and/or comprises a 3D Koosh ball trajectory.

8. A radiotherapy device (100) according to any preceding claim, wherein the controller being configured to generate a beam gating control signal based on the central k-space data comprises the controller being configured to determine a self-gating signal (302) based on the central k-space data, being configured to determine whether the self-gating signal is within a gating window (304), and being configured to generate the beam gating control signal in response to determining that the self-gating signal is not within the gating window.

9. A radiotherapy device (100) according to claim 8, wherein the controller is configured to determine projection data by applying a one-dimensional inverse fast Fourier transform to the central k-space data, and to determine the self-gating signal (302) based on the projection data.

10. A radiotherapy device (100) according to claim 9, wherein the controller is configured to determine the self-gating signal (302) by performing principal component analysis on the projection data.

11. A radiotherapy device (100) according to claim 9 or claim 10, wherein the self-gating signal (302) comprises a first self-gating signal determined by the controller for a training time period, the projection data comprising first projection data determined by the controller for the training time period, and wherein the controller is configured to determine a second self-gating signal for a subsequent time period by concatenating the first projection data for the training time period with second projection data for the subsequent time period.

12. A radiotherapy device (100) according to any preceding claim, wherein the radiation source is configured to apply the radiotherapy beam (110) to a tumour in at least one of a liver, pancreas, heart or oesophagus of the subject.

13. A computer-readable medium (828) comprising computer-executable instructions (822) which, when executed by a processor (802), cause the processor to:

apply a 3D MR pulse sequence using an MR imaging apparatus (112) to obtain k-space data for a subject;

select central k-space data from the k-space data;
generate a beam gating control signal based on the central k-space data; and,
after generating the beam gating control signal, reconstruct an image of the subject based at least in part on the k-space data.

**Patentansprüche**

1. Strahlentherapievorrichtung (100), umfassend:

   eine Strahlungsquelle, die dazu ausgelegt ist, einen Strahlentherapiestrahl (110) zum Bestrahlen eines Subjekts zu generieren;
   eine MR-Bildgebungseinrichtung (112), die dazu ausgelegt ist, eine 3D-MR-Pulssequenz anzuwenden, um k-Raum-Daten für das Subjekt zu erhalten; und
   eine Steuerung, die kommunikativ mit der Strahlungsquelle und der MR-Bildgebungseinrichtung gekoppelt ist, wobei die Steuerung ausgelegt ist zum:

      Auswählen zentraler k-Raum-Daten aus den k-Raum-Daten;
      Generieren eines Strahl-Gating-Steuersignals basierend auf den zentralen k-Raum-Daten; und
      nach dem Generieren des Strahl-Gating-Steuersignals Rekonstruieren eines Bildes des Subjekts mindestens teilweise basierend auf den k-Raum-Daten.

2. Strahlentherapievorrichtung (100) nach Anspruch 1, wobei die Steuerung dazu ausgelegt ist, das Strahl-Gating-Steuersignal unabhängig von Rekonstruieren eines Bildes des Subjekts mindestens teilweise basierend auf den k-Raum-Daten zu generieren.

3. Strahlentherapievorrichtung (100) nach Anspruch 1 oder Anspruch 2, wobei die Steuerung dazu ausgelegt ist, das Strahl-Gating-Steuersignal an die Strahlungsquelle zu übertragen, und die Strahlungsquelle dazu ausgelegt ist, den Strahlentherapiestrahl (110) basierend auf dem Strahl-Gating-Steuersignal zeitlich zu koppeln ("Gating" zu unterziehen).

4. Strahlentherapievorrichtung (100) nach einem der vorhergehenden Ansprüche, wobei die zentralen k-Raum-Daten ein einzelnes Pixel in der Mitte des k-Raums umfassen und/oder wobei die zentralen k-Raum-Daten eine Vielzahl von Pixeln in der Mitte des k-Raums und diese umgebend umfassen.

5. Strahlentherapievorrichtung (100) nach einem der vorhergehenden Ansprüche, wobei die MR-Bildgebungseinrichtung (112) dazu ausgelegt ist, zusätzliche k-Raum-Daten zu erhalten, und wobei die Steuerung dazu ausgelegt ist, das rekonstruierte Bild des Subjekts mindestens teilweise basierend auf den zusätzlichen k-Raum-Daten zu aktualisieren.

6. Strahlentherapievorrichtung (100) nach einem der vorhergehenden Ansprüche, wobei die 3D-MR-Pulssequenz dazu ausgelegt ist, die Mitte des k-Raums als Teil seiner k-Raum-Trajektorie wiederholt abzutasten.

7. Strahlentherapievorrichtung (100) nach einem der vorhergehenden Ansprüche, wobei die 3D-MR-Pulssequenz eine radiale Stack-of-Stars-Abtasttrajektorie umfasst, wobei die 3D-MR-Pulssequenz dazu vorgesehen ist, die Mitte des k-Raums durch Abtasten entlang einer Vielzahl von Speichen wiederholt abzutasten, wobei jede der Speichen um ein Winkelinkrement relativ zu einer vorherigen Speiche gedreht wird, und/oder eine Stack-of-Spirals-Abtasttrajektorie umfasst, und/oder eine 3D-Koosh-Ball-Trajektorie umfasst.

8. Strahlentherapievorrichtung (100) nach einem der vorhergehenden Ansprüche, wobei, dass die Steuerung dazu ausgelegt ist, ein Strahl-Gating-Steuersignal basierend auf den zentralen k-Raum-Daten zu generieren, umfasst, dass die Steuerung dazu ausgelegt ist, ein Selbst-Gating-Signal (302) basierend auf den zentralen k-Raum-Daten zu bestimmen, das dazu ausgelegt ist, zu bestimmen, ob das Selbst-Gating-Signal innerhalb eines Gating-Fensters (304) liegt, und dazu ausgelegt ist, das Strahl-Gating-Steuersignal als Reaktion auf Bestimmen, dass das Selbst-Gating-Signal nicht innerhalb des Gating-Fensters liegt, zu generieren.

9. Strahlentherapievorrichtung (100) nach Anspruch 8, wobei die Steuerung dazu ausgelegt ist, Projektionsdaten durch Anwenden einer eindimensionalen inversen Fast-Fourier-Transformation auf die zentralen k-Raum-Daten zu bestimmen, und das Selbst-Gating-Signal (302) basierend auf den Projektionsdaten zu bestimmen.

10. Strahlentherapievorrichtung (100) nach Anspruch 9, wobei die Steuerung dazu ausgelegt ist, das Selbst-Gating-Signal (302) durch Durchführen einer Hauptkomponentenanalyse an den Projektionsdaten zu bestimmen.

11. Strahlentherapievorrichtung (100) nach Anspruch 9 oder Anspruch 10, wobei das Selbst-Gating-Signal (302) ein erstes Selbst-Gating-Signal umfasst, das durch die Steuerung für einen Trainingszeitraum bestimmt wird, wobei die Projektionsdaten erste Projektionsdaten umfassen, die durch die Steuerung für den Trainingszeitraum bestimmt werden, und wobei die Steuerung dazu ausgelegt ist, ein zweites Selbst-Gating-Signal für einen nachfolgenden Zeitraum durch Verketten der ersten Projektionsdaten für den Trainingszeitraum mit zweiten Projektionsdaten für den nachfolgenden Zeitraum zu bestimmen.

12. Strahlentherapievorrichtung (100) nach einem der vorhergehenden Ansprüche, wobei die Strahlungsquelle dazu ausgelegt ist, den Strahlentherapiestrahl (110) auf einen Tumor in mindestens einem von Leber, Pankreas, Herz oder Ösophagus des Subjekts anzuwenden.

13. Computerlesbares Medium (828), umfassend computerausführbare Anweisungen (822), die bei Ausführung durch einen Prozessor (802) bewirken, dass der Prozessor Folgendes ausführt:

Anwenden einer 3D-MR-Pulssequenz unter Verwendung einer MR-Bildgebungseinrichtung (112), um k-Raum-Daten für ein Subjekt zu erhalten;
Auswählen zentraler k-Raum-Daten aus den k-Raum-Daten;
Generieren eines Strahl-Gating-Steuersignals basierend auf den zentralen k-Raum-Daten; und
nach dem Generieren des Strahl-Gating-Steuersignals Rekonstruieren eines Bildes des Subjekts mindestens teilweise basierend auf den k-Raum-Daten.

**Revendications**

1. Dispositif de radiothérapie (100) comprenant :

une source de rayonnement configurée pour générer un faisceau de radiothérapie (110) pour exposer un sujet à un rayonnement ;
un appareil d'imagerie par résonance magnétique (112) configuré pour appliquer une séquence d'impulsions RM 3D afin d'obtenir des données d'espace k pour le sujet ; et
un dispositif de commande couplé en communication à la source de rayonnement et à l'appareil d'imagerie par résonance magnétique, le dispositif de commande étant configuré pour :

sélectionner des données centrales d'espace k à partir des données d'espace k ;
générer un signal de commande de synchronisation de faisceau basé sur les données centrales d'espace k ; et
après génération du signal de commande de synchronisation de faisceau, reconstruire une image du sujet sur la base au moins en partie des données d'espace k.

2. Dispositif de radiothérapie (100) selon la revendication 1, dans lequel le dispositif de commande est configuré pour générer le signal de commande de synchronisation de faisceau indépendamment de la reconstruction d'une image du sujet sur la base au moins en partie des données d'espace k.

3. Dispositif de radiothérapie (100) selon la revendication 1 ou la revendication 2, dans lequel le dispositif de commande est configuré pour transmettre le signal de commande de synchronisation de faisceau à la source de rayonnement, et la source de rayonnement est configurée pour synchroniser le faisceau de radiothérapie (110) sur la base du signal de commande de synchronisation de faisceau.

4. Dispositif de radiothérapie (100) selon une quelconque revendication précédente, dans lequel les données centrales d'espace k comprennent un pixel unique au centre de l'espace k, et/ou dans lequel les données centrales d'espace k comprennent une pluralité de pixels au centre de l'espace k et autour de celui-ci.

5. Dispositif de radiothérapie (100) selon une quelconque revendication précédente, dans lequel l'appareil d'imagerie RM (112) est configuré pour obtenir des données d'espace k supplémentaires, et dans lequel le dispositif de commande est configuré pour mettre à jour l'image reconstruite du sujet sur la base au moins en partie des données d'espace k supplémentaires.

6. Dispositif de radiothérapie (100) selon une quelconque revendication précédente, dans lequel la séquence d'impulsions RM 3D est conçue pour échantillonner de manière répétitive le centre de l'espace k en tant que partie de sa trajectoire d'espace k.

7. Dispositif de radiothérapie (100) selon une quelconque revendication précédente, dans lequel la séquence d'impulsions RM 3D comprend une trajectoire d'échantillonnage en empilement d'étoiles radial dans laquelle la séquence d'impulsions MR 3D est conçue pour échantillonner de manière répétitive le centre de l'espace k par échantillonnage le long d'une pluralité de rayons, chacun des rayons étant tourné d'un incrément angulaire par rapport à un rayon précédent, et/ou comprend une trajectoire d'échantillonnage en empilement de spirales, et/ou comprend une trajectoire en balle Koosh 3D.

8. Dispositif de radiothérapie (100) selon une quelconque revendication précédente, dans lequel le dispositif de commande étant configuré pour générer un signal de commande de synchronisation de faisceau sur la base des données centrales d'espace k comprend le dispositif de commande étant configuré pour déterminer un signal d'autosynchronisation (302) sur la base des données centrales d'espace k, étant configuré pour déterminer si le signal d'autosynchronisation se trouve à l'intérieur d'une fenêtre de synchronisation (304), et étant configuré pour générer le signal de commande de synchronisation de faisceau en réponse à la détermination du fait que le signal d'autosynchronisation ne se trouve pas à l'intérieur de la fenêtre de synchronisation.

9. Dispositif de radiothérapie (100) selon la revendication 8, dans lequel le dispositif de commande est configuré pour déterminer des données de projection en appliquant une transformée de Fourier rapide inverse unidimensionnelle aux données centrales d'espace k, et pour déterminer le signal d'autosynchronisation (302) sur la base des données de projection.

10. Dispositif de radiothérapie (100) selon la revendication 9, dans lequel le dispositif de commande est configuré pour déterminer le signal d'autosynchronisation (302) en effectuant une analyse de composante principale sur les données de projection.

11. Dispositif de radiothérapie (100) selon la revendication 9 ou la revendication 10, dans lequel le signal d'autosynchronisation (302) comprend un premier signal d'autosynchronisation déterminé par le dispositif de commande pour une période d'entraînement, les données de projection comprenant des premières données de projection déterminées par le dispositif de commande pour la période d'entraînement, et dans lequel le dispositif de commande est configuré pour déterminer un second signal d'autosynchronisation pour une période suivante en concaténant les premières données de projection pour la période d'entraînement avec les secondes données de projection pour la période suivante.

12. Dispositif de radiothérapie (100) selon une quelconque revendication précédente, dans lequel la source de rayonnement est configurée pour appliquer le faisceau de radiothérapie (110) à une tumeur dans le foie et/ou le pancréas et/ou le cœur et/ou l'œsophage du sujet.

13. Support lisible par ordinateur (828) comprenant des instructions exécutables par ordinateur (822) qui, lorsqu'elles sont exécutées par un processeur (802), amènent le processeur à :

   appliquer une séquence d'impulsions RM 3D à l'aide d'un appareil d'imagerie RM (112) pour obtenir des données d'espace k pour un sujet ;
   sélectionner des données centrales d'espace k à partir des données d'espace k ;
   générer un signal de commande de synchronisation de faisceau basé sur les données centrales d'espace k ; et
   après génération du signal de commande de synchronisation de faisceau, reconstruire une image du sujet sur la base au moins en partie des données d'espace k.

100

116

102

118

104

106

108

110

112

114

Fig. 1

Fig. 2

Fig. 3a

Gating simulation: $\alpha = 0.8$, $\beta = 1.0$, $t_a = 4$, $t_p = 10$

302

Fig. 3b

Gating simulation: $\alpha = 0.8$, $\beta = 1.0$, $t_a = 8$, $t_p = 10$

Fig. 4

500

502
Apply a 3D MR pulse sequence to obtain k-space data for the subject over an acquisition period of $t_p$ seconds

504
Calculate absolute projection data from the k-space data

506
Determine the self-gating signal for each receiver coil using principal component analysis. Save the eigenvectors $\Psi_{tp}$

508
Combine the self-gating signals from all receiver coils. Save the coil selection vector $CC_{tp}$

510
Determine the normalized self-gating signal. Save the normalization coefficients $A_{tp}$ and $B_{tp}$

# Fig. 5

600

**602**
Apply a 3D MR pulse sequence to obtain k-space data for the subject over an acquisition period of $t_p$ seconds

**604**
Determine the normalized self-gating signal according to the steps defined in 504 to 510.

606

**608**
Apply a 3D MR pulse sequence to obtain k-space data for the subject over an acquisition period of $t > t_p$ seconds

**610**
Calculate absolute projection data from the k-space data

**612**
Determine the self-gating signal for each receiver coil by applying the eigenvectors $\Psi_{tp}$

**614**
Combine the self-gating signals from all receiver coils using the coil selection vector $CC_{tp}$

**616**
Determine the normalized self-gating signal using the normalization coefficients $A_{tp}$ and $B_{tp}$

Fig. 6

700

702
Generate a radiotherapy beam for irradiating a subject

704
Apply a 3D MR pulse sequence to obtain k-space data for the subject

706
Determine a self-gating signal based on central k-space data selected from the k-space data

708
Is the self-gating signal within a gating window?

No

Yes

710
Generate a beam gating control signal for gating the radiotherapy beam

712
Reconstruct an image of the subject based on the k-space data

Fig. 7

EP 4 347 014 B1

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020219814 A1

- DE 102013206315 A1